# EUROPEAN PATENT SPECIFICATION

(11) **EP 1 768 999 B1**
(45) Date of publication and mention of the grant of the patent: **19.06.2013**
(21) Application number: 05789212.7
(22) Date of filing: 30.06.2005
(51) Int. Cl.: C07K 14/47, C07H 21/04, A61K 38/17, A61K 48/00

(54) **sHIgM12 antibody useful to treat multiple sclerosis**
Antikörper sHIgM12 zur Behandlung von Multiple-Sklerose
Anticorps sHIgM12 pour le traitement de la sclérose en plaques

(30) Priority: 30.06.2004 US 881661; 05.11.2004 US 983104
(43) Date of publication of application: 04.04.2007
(60) Divisional application: 12164916.4
(73) Proprietor: MAYO FOUNDATION FOR MEDICAL EDUCATION AND RESEARCH, Rochester, Minnesota 55905 (US)
(72) Inventor: PEASE, Larry R., Rochester, MN 55906 (US); RADHAKRISHNAN, Suresh, Rochester, MN 55901 (US); VAN KEULEN, Virginia, Rochester, MN 55904 (US); CIRIC, Bogoljub, Philadelphia, PA 19103 (US); IIJIMA, Koji, Rochester, MN 55904 (US); KITA, Hirohito, Rochester, MN 55901 (US)
(74) Representative: Grund, Martin
(86) International application number: PCT/US2005/023440
(87) International publication number: WO 2006/004988

(56) References cited:
- EP-A- 1 297 847
- EP-A- 1 391 464
- WO-A-01/62932
- WO-A-92/03569
- WO-A-99/33965
- WO-A-03/064992
- WO-A-2004/050683
- JP-A- 9 100 300
- JP-A- 11 127 855
- US-A1- 2002 091 246
- US-A1- 2002 110 836
- US-A1- 2003 211 100
- MATSUMOTO KOICHIRO ET AL: "B7-DC regulates asthmatic response by an IFN-gamma-dependent mechanism." JOURNAL OF IMMUNOLOGY, vol. 172, no. 4, 15 February 2004 (2004-02-15), pages 2530-2541, XP002491039 ISSN: 0022-1767
- NGUYEN LOC T ET AL: "Cross-linking the B7 family molecule B7-DC directly activates immune functions of dendritic cells." JOURNAL OF EXPERIMENTAL MEDICINE, vol. 196, no. 10, 18 November 2002 (2002-11-18), pages 1393-1398, XP002491040 ISSN: 0022-1007
- RADHAKRISHNAN SURESH ET AL: "Naturally occurring human IgM antibody that binds B7-DC and potentiates T cell stimulation by dendritic cells." JOURNAL OF IMMUNOLOGY, vol. 170, no. 4, 15 February 2003 (2003-02-15), pages 1830-1838, XP002491041 ISSN: 0022-1767
- RADHAKRISHNAN SURESH ET AL: "Blockade of allergic airway inflammation following systemic treatment with a B7-dendritic cell (PD-L2) cross-linking human antibody" JOURNAL OF IMMUNOLOGY, vol. 173, no. 2, 15 July 2004 (2004-07-15), pages 1360-1365, 1355, XP002491042 ISSN: 0022-1767
- RADHAKRISHNAN SURESH ET AL: "Immunotherapeutic potential of B7-DC (PD-L2) cross-linking antibody in conferring antitumor immunity" CANCER RESEARCH, vol. 64, no. 14, 15 July 2004 (2004-07-15), pages 4965-4972, XP002491043 ISSN: 0008-5472
- RADHAKRISHNAN ET AL: "Dendritic cells activated by cross-linking B7-DC (PD-L2) block inflammatory airway disease" JOURNAL OF ALLERGY AND CLINICAL IMMUNOLOGY, MOSBY - YEARLY BOOK, INC, US, vol. 116, no. 3, 1 September 2005 (2005-09-01), pages 668-674, XP005068201 ISSN: 0091-6749
- KLEIN R. ET AL: 'Expressed human immunoglobulin-kappa genes and their hypermutation' EUR. J. IMMUNOL. vol. 23, 1993, pages 3248 - 3262, XP002971863
- DENIC ALEKSANDAR ET AL: "A single dose of neuron-binding human monoclonal antibody improves spontaneous activity in a murine model of demyelination.", PLOS ONE 2011, vol. 6, no. 10, 2011, page e26001, ISSN: 1932-6203
- WRIGHT BRENT R ET AL: "Cellular mechanisms of central nervous system repair by natural autoreactive monoclonal antibodies.", ARCHIVES OF NEUROLOGY DEC 2009, vol. 66, no. 12, December 2009 (2009-12), pages 1456-1459, ISSN: 1538-3687
- SCHWARTZ-ALBIEZ R ET AL: "Natural antibodies, intravenous immunoglobulin and their role in autoimmunity, cancer and inflammation.", CLINICAL AND EXPERIMENTAL IMMUNOLOGY DEC 2009, vol. 158 Suppl 1, December 2009 (2009-12), pages 43-50, ISSN: 1365-2249
- WARRINGTON ARTHUR E ET AL: "Neuron-binding human monoclonal antibodies support central nervous system neurite extension", JOURNAL OF NEUROPATHOLOGY AND EXPERIMENTAL NEUROLOGY, LIPPINCOTT WILLIAMS AND WILKINS, NEW YORK, NY, vol. 63, no. 5, 1 May 2004 (2004-05-01), pages 461-473, XP009165099, ISSN: 0022-3069

## Description

### TECHNICAL FIELD

This document relates to an antibody.

### BACKGROUND

Decavalent IgM antibodies display measurable binding avidity to antigens, even though binding affinity may be low. The multivalent structure of pentameric IgM provides the potential for cross-linking cell surface targets, endowing the soluble antibodies with biological potential not normally associated with immune function.

Dendritic cells (DC) are efficient antigen-presenting cells (APC). These cells express class I and class II major histocompatibility complex (MHC) peptide-presenting molecules on their cell surfaces, along with a series of costimulatory molecules (Banchereau and Steinman (1998) Nature 392:245-252). Naive T cells express receptors for these DC ligands. Following recognition of peptide-antigen presented in the context of class I or class II molecules, the structure of the T cell membrane is reorganized, bringing together the elements of the T cell receptor with other cell-surface molecules, including the co-receptors CD4 or CD8 and the costimulatory receptors CD28 and CTLA-4 (Monks et al. (1998) Nature 395:82-86; and Wulfing and Davis (1998) Science 282:2266-2269). Interactions within the newly formed macromolecular complexes determine the outcome of inductive events transduced into T cells by DC.

DC reside in a variety of tissues and display distinct tissue-associated phenotypes (Strunk et al. (1997) J. Exp. Med. 185:1131-1136; Caux et al. (1996) J. Exp. Med. 184:695-706; Wu et al. (1996) J. Exp. Med. 184:903-911; and Vremec et al. (1992) J. Exp. Med. 176:47-58). The relationships among the cell lineages of these different subsets of cells are not firmly established. A large body of work has emerged focusing on DC generated *in vitro* from bone marrow or blood precursors (Mayordomo et al. (1995) Nat. Med. 1:1297-1302; Nonacs et al. (1992) J. Exp. Med. 176:519-529; Steinman and Witmer (1978) Proc. Natl. Acad. Sci. USA 75:5132-5136; and Young and Steinman (1990) J. Exp. Med 171:1315-1332). The cells generated *in vitro* express high levels of class I antigens and the series of costimulatory ligands associated with endogenous DC (Fagnoni et al. (1995) Immunology 85:467-474; and Banchereau et al. (2000) Annu. Rev Immunol. 18:767-811). Importantly, they are able to efficiently activate naive T cells, a function that is the signature of the DC.

Asthma is primarily a chronic inflammatory disease of the airways. This primary inflammation causes two secondary symptoms: (a) overly reactive bronchi that are more sensitive to various asthma triggers such as allergens, cold and dry air, smoke and viruses, and (b) airflow obstruction (i.e., difficulty moving air in and out of the lungs). These symptoms are typically manifested by coughing, wheezing, shortness of breath or rapid breathing, and chest tightness.

Allergic asthma is the most common form of asthma. Many of the symptoms of allergic and non-allergic asthma are the same. Allergic asthma is triggered by inhaling allergens such as dust mites, pet dander, pollens, or mold. Through a complex reaction, these allergens then cause the passages in the airways of the lungs to become inflamed and swollen, resulting in asthma symptoms.

Allergic asthma is characterized by pulmonary inflammatory infiltration and hyperreactivity to variety of lung irritants and stimuli such as methacholine. The hallmark of allergic asthma is abnormal expansion of Th2 cells in the lungs (Wills-Karp (1999) Annu. Rev. Immunol. 17:255-281; and Ray and Cohn (1999) J. Clin. Invest. 104:985-993). DC act as the major antigen presenting cells to naïve T cells in lymphoid organs (Steinman (1991) Annu. Rev. Immunol. 9:271-296). DC are present in the respiratory tract, and upon isolation from the trachea, bronchi, alveoli and visceral pleura, they are capable of antigen presentation to T cells (Holt and Schon-Hegrad (1987) Immunol. 62:349-356; and Sertl et al. (1986) J. Exp. Med. 163:436-451). In addition, it has been demonstrated using an ovalbumin (OVA) model of allergic asthma that pulmonary DC prime T cells, inducing a Th2 phenotype. Treatment of mice with GM-CSF modulated this effect toward a Th1 polarity, with an accompanying increase in message for IL-12 (Stumbles et al. (1998) J. Exp. Med. 188:2019-2031).

### SUMMARY

As such, provided herein are polypeptides having amino acid sequences that are similar or identical to the amino acid sequence of the sHIgM12 antibody. Also provided herein are nucleic acid molecules encoding polypeptides that have amino acid sequences similar or identical to the amino acid sequence of sHIgM12. Methods provided herein can be useful to treat or reduce development of conditions involving a pathological immune response (e.g., irritable bowel disease or multiple sclerosis).

In one aspect, this document features a purified polypeptide containing an amino acid sequence that is at least 95.0% (e.g., 99.1% or 99.2%) identical to the amino acid sequence set forth in SEQ ID NO:6 or SEQ ID NO:8. The purified polypeptide can further contain an amino acid sequence that is at least 80.0% identical to the amino acid sequence set forth in SEQ ID NO:7, SEQ ID NO:9, SEQ ID NO:10, SEQ ID NO:11, or SEQ ID NO:12.

In another aspect, this document features an isolated nucleic acid encoding a polypeptide that comprises an amino acid sequence that is at least 95.0% (e.g., 99.1% or 99.2%) identical to the amino acid sequence set forth in SEQ ID NO:6 or SEQ ID NO:8. The encoded polypeptide can further contain an amino acid sequence that is between 80.0% and 99.9% identical to the amino acid sequence set forth in SEQ ID NO:7, SEQ ID NO:9, SEQ ID NO:10, SEQ ID NO:11, or SEQ ID NO:12.

In another aspect, this document features a composition containing a polypeptide and a pharmaceutically acceptable carrier, wherein the polypeptide contains an amino acid sequence that is between 95% and 99.9% identical to the amino acid sequence set forth in SEQ ID NO:6 or SEQ ID NO:8. The polypeptide can further contain an amino acid sequence that is between 80.0% and 99.9% identical to the amino acid sequence set forth in SEQ ID NO:7, SEQ ID NO:9, SEQ ID NO:10, SEQ ID NO:11, or SEQ ID NO:12.

In still another aspect, this document features a composition containing a nucleic acid molecule and a pharmaceutically acceptable carrier, wherein the nucleic acid encodes a polypeptide containing an amino acid sequence that is between 95% and 99.9% identical to the amino acid sequence set forth in SEQ ID NO:6 or SEQ ID NO:8. The encoded polypeptide can further contain an amino acid sequence that is between 80.0% and 99.9% identical to the amino acid sequence set forth in SEQ ID NO:7, SEQ ID NO:9, SEQ ID NO:10, SEQ ID NO:11, or SEQ ID NO:12.

In another aspect, this document features an isolated nucleic acid molecule containing a nucleotide sequence that is the nucleotide sequence set forth in SEQ ID NO:13 or SEQ ID NO:14.

Unless otherwise defined, all technical and scientific terms used herein have the same meaning as commonly understood by one of ordinary skill in the art to which this invention pertains. Although methods and materials similar or equivalent to those described herein can be used to practice the invention, suitable methods and materials are described below. In addition, the materials, methods, and examples are illustrative only and not intended to be limiting.

The details of one or more embodiments of the invention are set forth in the accompanying drawings and the description below. Other features, objects, and advantages of the invention will be apparent from the description and drawings, and from the claims.

### DESCRIPTION OF DRAWINGS

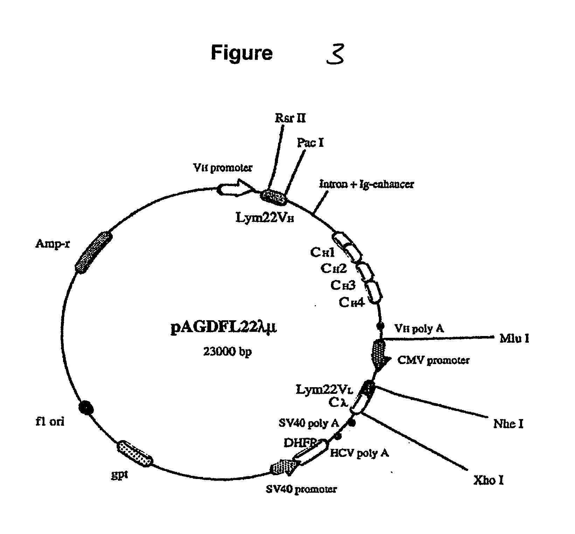

FIG. 3 is a map of an expression vector that can be used to produce antibodies.
FIG 1A shows the amino acid sequences of the variable (Vk) and constant (Ck) domains (SEQ ID NOS:6 and 7, respectively) of the sHIgM12 light chain. FIG. 1B shows the amino acid sequences of the variable (Vh) and constant (CH1, CH2, CH3, CH4) domains (SEQ ID NOS:8, 9, 10, 11, and 12, respectively) of the sHIgM12 heavy chain.
FIG 2 shows nucleic acid sequences that encode the Vk and Vh domains (SEQ ID NOS:13 and 14, respectively) of sHIgM12.

### DETAILED DESCRIPTION

Provided herein are polypeptides containing an amino acid sequence that is similar or identical to an amino acid sequence present within the sHIgM12 antibody. Also provided herein are nucleic acid molecules encoding polypeptides that contain an amino acid sequence similar or identical to the amino acid sequence of the sHIgM12 antibody.

### 1. Molecules

The molecules provided herein typically are purified. The term "purified" as used herein refers to a molecule that has been separated or isolated from other cellular components by which it is naturally accompanied (e.g., other cellular proteins, polynucleotides, or cellular components), or separated from most or all other components present in a reaction mixture when the molecule is synthesized *in vitro.* "Purified" as used herein also encompasses molecules that are partially purified, so that at least some of the components by which the molecule is accompanied are removed. Typically, a molecule is considered "purified" when it is at least 50% (e.g., 55%, 60%, 70%, 80%, 90%, 95%, or 99%), by dry weight, free from the proteins and other organic molecules or components with which it naturally associates or with which it is accompanied in a synthesis reaction.

### 2. Polypeptides and Antibodies

Molecules provided herein are polypeptides. As used herein, a polypeptide is an amino acid chain, regardless of length or post-translational modification (e.g., phosphorylation or glycosylation).

The polypeptides featured herein contain an amino acid sequence that is similar or identical to the amino sequence of sHIgM12. For example, a polypeptide can contain an amino acid sequence that is at least 95% identical (e.g. 95.0%, 97.0%, 97.5%, 98.0%, 98.5%, 99.0%, 99.1%, 99.2%, 99.3%, 99.4%, 99.5%, 99.6%, 99.7%, 99.8%, or 99.9% identical) to the amino acid sequence set forth in SEQ ID NO 6 or SEQ ID NO:8. In some embodiments, a polypeptide can further contain an amino acid sequence that is at least 80.0% identical (e.g., 80.0%, 85.0%, 90.0%, 95.0%, 97.0%, 97.5%, 98.0%, 98.5%, 99.0%, 99.1%, 99.2%, 99.3%, 99.4%, 99.5%, 99.6%, 99.7%, 99.8%, or 99.9% identical) to the amino acid sequence set forth in SEQ ID NO:7, SEQ ID NO:9, SEQ ID NO:10, SEQ ID NO:11, or SEQ ID NO:12. Percent sequence identity is calculated by determining the number of matched positions in aligned nucleic acid sequences, dividing the number of matched positions by the total number of aligned nucleotides, and multiplying by 100. A matched position refers to a position in which identical nucleotides occur at the same position in aligned nucleic acid sequences. Percent sequence identity also can be determined for any amino acid sequence.

To determine percent sequence identity, a target nucleic acid or amino acid sequence is compared to the identified nucleic acid or amino acid sequence using the BLAST 2 Sequences (B12seq) program from the stand-alone version of BLASTZ containing BLASTN version 2.0.14 and BLASTP version 2.0.14. This stand-alone version of BLASTZ can be obtained from Fish & Richardson's web site (World Wide Web at fr.com/blast) or the U.S. government's National Center for Biotechnology Information web site (World Wide Web at ncbi.nlm.nih.gov). Instructions explaining how to use the B12seq program can be found in the readme file accompanying BLASTZ.

B12seq performs a comparison between two sequences using either the BLASTN or BLASTP algorithm. BLASTN is used to compare nucleic acid sequences, while BLASTP is used to compare amino acid sequences. To compare two nucleic acid sequences, the options are set as follows: -i is set to a file containing the first nucleic acid sequence to be compared (e.g., C:\seq1.txt); -j is set to a file containing the second nucleic acid sequence to be compared (e.g., C:\seq2.txt); -p is set to blastn; -o is set to any desired file name (e.g., C:\output.txt); -q is set to -1; -r is set to 2; and all other options are left at their default setting. For example, the following command can be used to generate an output file containing a comparison between two sequences: C:\Bl2seq -i c:\seq1.txt-j c:\seq2.txt -p blastn -o c:\output.txt -q -1-r 2. To compare two amino acid sequences, the options of B12seq are set as follows: -i is set to a file containing the first amino acid sequence to be compared (e.g., C:\seq1.txt); -j is set to a file containing the second amino acid sequence to be compared (e.g., C:\seq2.txt); -p is set to blastp; -o is set to any desired file name (e.g., C:\output.txt); and all other options are left at their default setting. For example, the following command can be used to generate an output file containing a comparison between two amino acid sequences: C:\B12seq -i c:\seq1.txt -j c:\Seq2.txt -p blastp -o c:\output.txt. If the two compared sequences share homology, then the designated output file will present those regions of homology as aligned sequences. If the two compared sequences do not share homology, then the designated output file will not present aligned sequences.

Once aligned, the number of matches is determined by counting the number of positions where an identical nucleotide or amino acid residue is presented in both sequences. The percent sequence identity is determined by dividing the number of matches either by the length of the sequence set forth in the identified sequence (e.g., SEQ ID NO:6), or by an articulated length (e.g., 100 consecutive nucleotides or amino acid residues from a sequence set forth in an identified sequence), followed by multiplying the resulting value by 100. For example, a nucleic acid sequence that has 98 matches when aligned with the sequence set forth in SEQ ID NO:6 is 92.5 percent identical to the sequence set forth in SEQ ID NO:6 (i.e., 98÷106*100=92.5). It is noted that the percent sequence identity value is rounded to the nearest tenth. For example, 75.11, 75.12, 75.13, and 75.14 is rounded down to 75.1, while 75.15, 75.16, 75.17, 75.18, and 75.19 is rounded up to 75.2. It is also noted that the length value will always be an integer.

The amino acid sequences of the polypeptides provided herein can have substitutions, deletions, or additions with respect to the amino acid sequences set forth in SEQ ID NOS:6 and 8. A polypeptide having an amino acid sequence that is modified (e.g., by substitution) with respect to SEQ ID NO:6 and/or SEQ ID NO:8 can have substantially the same or improved qualities as compared to a polypeptide containing the amino acid sequence identical to that set forth in SEQ ID NO:6 and SEQ ID NO:8. A substitution can be a conserved substitution. As used herein, a "conserved substitution" is a substitution of an amino acid with another amino acid having a similar side chain. A conserved substitution typically can be a substitution with an amino acid that makes the smallest change possible in the charge of the amino acid or size of the side chain of the amino acid (alternatively, in the size, charge or kind of chemical group within the side chain) such that the overall peptide essentially retains its spatial conformation but has altered biological activity. Examples of conserved changes include, without limitation, Asp to Glu, Asn or Gln; His to Lys, Arg or Phe; Asn to Gln, Asp or Glu, and Ser to Cys, Thr or Gly. Alanine is commonly used to substitute for other amino acids. The 20 essential amino acids can be grouped as follows: alanine, valine, leucine, isoleucine, proline, phenylalanine, tryptophan and methionine having nonpolar side chains; glycine, serine, threonine, cysteine, tyrosine, asparagine and glutamine having uncharged polar side chains; aspartate and glutamate having acidic side chains; and lysine, arginine, and histidine having basic side chains (see, e.g., Stryer, Biochemistry (2nd edition) W. H. Freeman and Co. San Francisco (1981), pp. 14-15; and Lehninger, Biochemistry (2nd edition, 1975), pp. 73-75). Conservative substitutions can include substitutions made within these groups.

Molecules provided herein are antibodies. The terms "antibody" and "antibodies" encompass intact molecules as well as fragments thereof. Antibodies can be polyclonal antibodies, monoclonal antibodies, humanized or chimeric antibodies, single chain Fv antibody fragments, Fab fragments, and F(ab)₂ fragments. Polyclonal antibodies are heterogeneous populations of antibody molecules that are specific for a particular antigen, while monoclonal antibodies are homogeneous populations of antibodies to a particular epitope contained within an antigen.

An antibody can be of any immunoglobulin (Ig) class, including IgM, IgA, IgD, IgE, and IgG, and any subclass thereof. Antibodies of the IgM class (e.g., sHIgM12) typically are pentavalent and are particularly useful. Immune complexes containing Ig molecules that are cross-linked (e.g., cross-linked IgG) and are thus multivalent also can be particularly useful.

As used herein, an "epitope" is a portion of an antigenic molecule to which an antibody binds. Antigens can present more than one epitope at the same time. For polypeptide antigens, an epitope typically is about four to six amino acids in length, and can include modified (e.g., phosphorylated or glycosylated) amino acids. Two different immunoglobulin can have the same epitope specificity if they bind to the same epitope or set of epitopes.

Polyclonal antibodies are contained in the sera of immunized animals. Monoclonal antibodies can be prepared using, for example, standard hybridoma technology. In particular, monoclonal antibodies can be obtained by any technique that provides for the production of antibody molecules by continuous cell lines in culture as described, for example, by Kohler et al. (1975) Nature 256:495-497, the human B-cell hybridoma technique ofKosbor et al. (1983) Immunology Today 4:72, and Cote et al. (1983) Proc. Natl. Acad. Sci. USA 80:2026-2030, and the EBV-hybridoma technique of Cole et al., Monoclonal Antibodies and Cancer Therapy, Alan R Liss, Inc. pp. 77-96 (1983). A hybridoma producing monoclonal antibodies can be cultivated *in vitro* or *in vivo.*

Antibodies provided herein also can be isolated from, for example, the serum of an individual. The sIDgM12 antibody, for example, was isolated from human serum as described in Example 1 herein. Suitable methods for isolation include purification from mammalian serum using techniques that include, for example, chromatography.

Antibodies such as sHIgM12 also can be produced recombinantly. The amino acid sequence (e.g., the partial amino acid sequence) of an antibody provided herein can be determined by standard techniques, and a cDNA encoding the antibody or a portion of the antibody can be isolated from the serum of the subject (e.g., the human patient or the immunized host animal) from which the antibody was originally isolated. The cDNA can be cloned into an expression vector using standard techniques. The expression vector then can be transfected into an appropriate host cell (e.g., a Chinese hamster ovary cell, a COS cell, or a hybridoma cell), and the antibody can be expressed and purified. See, for example, Example 2 herein.

Antibody fragments that retain cross-linking function also can be generated by techniques such as those disclosed above. Such antibody fragments include, but are not limited to, F(ab')₂ fragments that can be produced by pepsin digestion of an antibody molecule, and Fab fragments that can be generated by reducing the disulfide bridges of F(ab')₂ fragments. Alternatively, Fab expression libraries can be constructed. See, for example, Huse et al. (1989) Science 246:1275-1281. Single chain Fv antibody fragments are formed by linking the heavy and light chain fragments of the Fv region via an amino acid bridge (e.g., 15 to 18 amino acids), resulting in a single chain polypeptide. Single chain Fv antibody fragments can be produced through standard techniques, such as those disclosed in U.S. Patent No. 4,946,778. Such fragments can be rendered multivalent by, for example, biotinylation and cross-linking.

### 3. Nucleic acids, vectors, and host cells

Nucleic acids encoding molecules (e.g., polypeptides and antibodies such as those described herein) are provided herein. As used herein, the term "nucleic acid" refers to both RNA and DNA, including cDNA, genomic DNA, and synthetic (e.g., chemically synthesized) DNA. A nucleic acid molecule can be double-stranded or single-stranded (i.e., a sense or an antisense single strand). Nucleic acids can include, for example, cDNAs encoding the light and heavy chains of the sHIgM12 antibody.

An "isolated nucleic acid" refers to a nucleic acid that is separated from other nucleic acid molecules that normally flank one or both sides of the nucleic acid in the genome in which it is normally found. The term "isolated" as used herein with respect to nucleic acids also includes any non-naturally-occurring nucleic acid sequence, since such non-naturally-occurring sequences are not found in nature and do not have immediately contiguous sequences in a naturally-occurring genome.

An isolated nucleic acid can be, for example, a DNA molecule, provided one of the nucleic acid sequences normally found immediately flanking that DNA molecule in its naturally-occurring genome is removed or absent. Thus, an isolated nucleic acid includes, without limitation, a DNA molecule that exists as a separate molecule (e.g., a chemically synthesized nucleic acid, or a cDNA or genomic DNA fragment produced by PCR or restriction endonuclease treatment) independent of other sequences as well as DNA that is incorporated into a vector, an autonomously replicating plasmid, a virus (e.g., a retrovirus, lentivirus, adenovirus, or herpes virus), or into the genomic DNA of a prokaryote or eukaryote. In addition, an isolated nucleic acid can include an engineered nucleic acid such as a DNA molecule that is part of a hybrid or fusion nucleic acid. A nucleic acid existing among hundreds to millions of other nucleic acids within, for example, cDNA libraries or genomic libraries, or gel slices containing a genomic DNA restriction digest, is not considered an isolated nucleic acid.

The isolated nucleic acids disclosed herein can encode polypeptides. For example, an isolated nucleic acid can encode a polypeptide containing an amino acid sequence that is similar or identical to an amino acid sequence found in the variable or constant regions of sHIgM12. In one embodiment, a nucleic acid can encode a polypeptide containing an amino acid sequence that is at least 95.0% (e.g., 95.0%, 97.0%, 97.5%, 98.0%, 98.5%, 99.0%, 99.1%, 99.2%, 99.3%, 99.4%, 99.5%, 99.6%, 99.7%, 99.8%, or 99.9%) identical to the amino acid sequence set forth in SEQ ID NO:6 or SEQ ID NO:8. The encoded polypeptide can further contain an amino acid sequence that is at least 80.0% (e.g., 80.0%, 85.0%, 90.0%, 95.0%, 97.0%, 97.5%, 98.0%, 98.5%, 99.0%, 99.1%, 99.2%, 99.3%, 99.4%, 99.5%, 99.6%, 99.7%, 99.8%, or 99.9%) identical to the amino acid sequence set forth in SEQ ID NO:7, SEQ ID NO:9, SEQ ID NO:10, SEQ ID NO:11, or SEQ ID NO:12. In another embodiment, an isolated nucleic acid can contain a nucleotide sequence that is identical to the nucleotide sequence set forth in SEQ ID NO:13 or SEQ ID NO:14. The method for determining percent sequence identity is provided herein.

The isolated nucleic acid molecules provided herein can be produced by standard techniques, including, without limitation, common molecular cloning and chemical nucleic acid synthesis techniques. For example, polymerase chain reaction (PCR) techniques can be used to obtain an isolated nucleic acid molecule encoding an antibody such as sHIgM12. Isolated nucleic acids also can be chemically synthesized, either as a single nucleic acid molecule (e.g., using automated DNA synthesis in the 3' to 5' direction using phosphoramidite technology) or as a series of polynucleotides. For example, one or more pairs of long polynucleotides (e.g., > 100 nucleotides) can be synthesized that contain the desired sequence, with each pair containing a short segment of complementarity (e.g., about 15 nucleotides) such that a duplex is formed when the polynucleotide pair is annealed. DNA polymerase is used to extend the polynucleotides, resulting in a single, double-stranded nucleic acid molecule per polynucleotide pair.

Also provided herein are vectors containing nucleic acids such as those described above. As used herein, a "vector" is a replicon, such as a plasmid, phage, or cosmid, into which another DNA segment may be inserted so as to bring about the replication of the inserted segment. The vectors provided herein can be expression vectors. An "expression vector" is a vector that includes one or more expression control sequences, and an "expression control sequence" is a DNA sequence that controls and regulates the transcription and/or translation of another DNA sequence.

In the expression vectors provided herein, a nucleic acid (e.g., a nucleic acid encoding the light and/or heavy chains of sHIgM12) is operably linked to one or more expression control sequences. As used herein, "operably linked" means incorporated into a genetic construct so that expression control sequences effectively control expression of a coding sequence of interest. Examples of expression control sequences include promoters, enhancers, and transcription terminating regions. A promoter is an expression control sequence composed of a region of a DNA molecule, typically within 100 to 500 nucleotides upstream of the point at which transcription starts (generally near the initiation site for RNA polymerase II). To bring a coding sequence under the control of a promoter, it is necessary to position the translation initiation site of the translational reading frame of the polypeptide between one and about fifty nucleotides downstream of the promoter. Enhancers provide expression specificity in terms of time, location, and level. Unlike promoters, enhancers can function when located at various distances from the transcription site. An enhancer also can be located downstream from the transcription initiation site. A coding sequence is "operably linked" and "under the control" of expression control sequences in a cell when RNApolymerase is able to transcribe the coding sequence into mRNA, which then can be translated into the protein encoded by the coding sequence. Expression vectors provided herein thus are useful to produce sHIgM12, as well as other molecules provided herein.

Suitable expression vectors include, without limitation, plasmids and viral vectors derived from, for example, bacteriophage, baculoviruses, tobacco mosaic virus, herpes viruses, cytomegalovirus, retroviruses, vaccinia viruses, adenoviruses, and adeno-associated viruses. Numerous vectors and expression systems are commercially available from such corporations as Novagen (Madison, WI), Clontech (Palo Alto, CA), Stratagene (La Jolla, CA), and Invitrogen/Life Technologies (Carlsbad, CA).

An expression vector can include a tag sequence designed to facilitate subsequent manipulation of the expressed nucleic acid sequence (e.g., purification or localization). Tag sequences, such as green fluorescent protein (GFP), glutathione S-transferase (GST), polyhistidine, c-myc, hemagglutinin, or Flag™ tag (Kodak, New Haven, CT) sequences typically are expressed as a fusion with the encoded polypeptide. Such tags can be inserted anywhere within the polypeptide including at either the carboxyl or amino terminus.

Host cells containing vectors as described herein also are provided. The term "host cell" is intended to include prokaryotic and eukaryotic cells into which a recombinant expression vector can be introduced. As used herein, "transformed" and "transfected" encompass the introduction of a nucleic acid molecule (e.g., a vector) into a cell by one of a number of techniques. Although not limited to a particular technique, a number of these techniques are well established within the art. Prokaryotic cells can be transformed with nucleic acids by, for example, electroporation or calcium chloride mediated transformation. Nucleic acids can be transfected into mammalian cells by techniques including, for example, calcium phosphate co-precipitation, DEAE-dextran-mediated transfection, lipofection, electroporation, or microinjection. Suitable methods for transforming and transfecting host cells are found in Sambrook et al., Molecular Cloning: A Laboratory Manual (2nd edition), Cold Spring Harbor Laboratory, New York (1989), and reagents for transformation and/or transfection are commercially available (e.g., Lipofectin^{®} (Invitrogen/Life Technologies); Fugene (Roche, Indianapolis, IN); and SuperFect (Qiagen, Valencia, CA)).

### 4. Compositions

The molecules described herein can be used in the manufacture of a medicament. Thus, the molecules described herein (e.g., antibodies such as sHIgM12) can be incorporated into compositions. Such compositions are provided herein.

Methods for formulating and subsequently administering therapeutic compositions are well known to those skilled in the art. Dosages typically are dependent on the responsiveness of the subject to the molecule, with the course of treatment lasting from several days to several months, or until a suitable immune response is achieved. Persons of ordinary skill in the art routinely determine optimum dosages, dosing methodologies and repetition rates. Optimum dosages can vary depending on the relative potency of an antibody, and generally can be estimated based on the EC₅₀ found to be effective in in vitro and/or in vivo animal models. Dosage typically is from 0.01 µg to 100 g per kg of body weight (e.g., from 1 µg to 100 mg, from 10 µg to 10 mg, or from 50 µg to 500 µg per kg of body weight). Compositions containing the molecules provided herein may be given once or more daily, weekly, monthly, or even less often.

In addition to the molecules provided herein, the compositions described herein further can contain antigens that will elicit a specific immune response. Suitable antigens include, for example, polypeptides or fragments of polypeptides expressed by tumors and pathogenic organisms. Killed viruses and bacteria, in addition to components of killed viruses and bacteria, also are useful antigens. Such antigens can stimulate immune responses against tumors or pathogens.

Compositions also can include DC that have been isolated from, for example, bone marrow, spleen, or thymus tissue. DC lines also can be useful in the compositions provided herein.

The molecules featured herein (e.g., antibodies such as sHIgM12) can be admixed, encapsulated, conjugated or otherwise associated with other molecules, molecular structures, or mixtures of compounds such as, for example, liposomes, receptor targeted molecules, or oral, topical or other formulations for assisting in uptake, distribution and/or absorption.

In some embodiments, a composition can contain a molecule provided herein (e.g., sHIgM12, a polypeptide containing an amino acid sequence that is at least 95.0% identical to SEQ ID NO:6 or SEQ ID NO:8 a nucleic acid encoding a polypeptide that contains an amino acid sequence at least 95.0% identical to the sequence set forth in SEQ ID NO:6 or SEQ ID NO:8, or a nucleic acid molecule containing a nucleotide sequence that is the sequence set forth in SEQ ID NO:13 or SEQ ID NO:14) in combination with a pharmaceutically acceptable carrier. Pharmaceutically acceptable carriers are pharmaceutically acceptable solvents, suspending agents, or any other pharmacologically inert vehicles for delivering antibodies to a subject. Pharmaceutically acceptable carriers can be liquid or solid, and can be selected with the planned manner of administration in mind so as to provide for the desired bulk, consistency, and other pertinent transport and chemical properties, when combined with one or more therapeutic compounds and any other components of a given pharmaceutical composition. Typical pharmaceutically acceptable carriers include, without limitation: water; saline solution; binding agents (e.g., polyvinylpyrrolidone or hydroxypropyl methylcellulose); fillers (e.g., lactose and other sugars, gelatin, or calcium sulfate); lubricants (e.g., starch, polyethylene glycol, or sodium acetate); disintegrates (e.g., starch or sodium starch glycolate); and wetting agents (e.g., sodium lauryl sulfate).

Pharmaceutical compositions containing molecules provided herein can be administered by a number of methods, depending upon whether local or systemic treatment is desired. Administration can be, for example, parenteral (e.g., by subcutaneous, intrathecal, intraventricular, intramuscular, or intraperitoneal injection, or by intravenous (i.v.) drip); oral; topical (e.g., transdermal, sublingual, ophthalmic, or intranasal); or pulmonary (e.g., by inhalation or insufflation of powders or aerosols). Administration can be rapid (e.g., by injection) or can occur over a period of time (e.g., by slow infusion or administration of slow release formulations). For administration to the central nervous system, antibodies can be injected or infused into the cerebrospinal fluid, typically with one or more agents capable of promoting penetration across the blood-brain barrier.

Compositions and formulations for parenteral, intrathecal or intraventricular administration include sterile aqueous solutions (e.g., sterile physiological saline), which also can contain buffers, diluents and other suitable additives (e.g., penetration enhancers, carrier compounds and other pharmaceutically acceptable carriers).

Compositions and formulations for oral administration include, for example, powders or granules, suspensions or solutions in water or non-aqueous media, capsules, sachets, or tablets. Such compositions also can incorporate thickeners, flavoring agents, diluents, emulsifiers, dispersing aids, or binders.

Formulations for topical administration include, for example, sterile and non-sterile aqueous solutions, non-aqueous solutions in common solvents such as alcohols, or solutions in liquid or solid oil bases. Such solutions also can contain buffers, diluents and other suitable additives. Pharmaceutical compositions and formulations for topical administration can include transdermal patches, ointments, lotions, creams, gels, drops, suppositories, sprays, liquids, and powders. Conventional pharmaceutical carriers, aqueous, powder or oily bases, thickeners and the like may be useful.

Pharmaceutical compositions include, but are not limited to, solutions, emulsions, aqueous suspensions, and liposome-containing formulations. These compositions can be generated from a variety of components that include, for example, preformed liquids, self-emulsifying solids and self emulsifying semisolids. Emulsion formulations are particularly useful for oral delivery of therapeutic compositions due to their ease of formulation and efficacy of solubilization, absorption, and bioavailability. Liposomes can be particularly useful due to their specificity and the duration of action they offer from the standpoint of drug delivery.

Molecules featured herein can encompass any pharmaceutically acceptable salts, esters, or salts of such esters, or any other compound which, upon administration to a subject, is capable of providing (directly or indirectly) the biologically active metabolite or residue thereof. Accordingly, for example, this document provides pharmaceutically acceptable salts of molecules such as antibodies (e.g., sHIgM12), prodrugs and pharmaceutically acceptable salts of such prodrugs, and other bioequivalents. A prodrug is a therapeutic agent that is prepared in an inactive form and is converted to an active form (i.e., drug) within the body or cells thereof by the action of endogenous enzymes or other chemicals and/or conditions. The term "pharmaceutically acceptable salts" refers to physiologically and pharmaceutically acceptable salts of the antibodies useful in methods described herein (i.e., salts that retain the desired biological activity of the parent antibodies without imparting undesired toxicological effects). Examples of pharmaceutically acceptable salts include, but are not limited to, salts formed with cations (e.g., sodium, potassium, calcium, or polyamines such as spermine); acid addition salts formed with inorganic acids (e.g., hydrochloric acid, hydrobromic acid, sulfuric acid, phosphoric acid, or nitric acid); salts formed with organic acids (e.g., acetic acid, citric acid, oxalic acid, palmitic acid, or fumaric acid); and salts formed with elemental anions (e.g., bromine, iodine, or chlorine).

Compositions additionally can contain other adjunct components conventionally found in pharmaceutical compositions. Thus, the compositions also can include compatible, pharmaceutically active materials such as, for example, antipruritics, astringents, local anesthetics or anti-inflammatory agents, or additional materials useful in physically formulating various dosage forms of the compositions provided herein, such as dyes, flavoring agents, preservatives, antioxidants, opacifiers, thickening agents, and stabilizers. Furthermore, the composition can be mixed with auxiliary agents, e.g., lubricants, preservatives, stabilizers, wetting agents, emulsifiers, salts for influencing osmotic pressure, buffers, colorings, flavorings, penetration enhancers, and aromatic substances. When added, however, such materials should not unduly interfere with the biological activities of the PNA components within the compositions provided herein.

Pharmaceutical formulations as disclosed herein, which can be presented conveniently in unit dosage form, can be prepared according to conventional techniques well known in the pharmaceutical industry. Such techniques include the step of bringing into association the active ingredients (i.e., the antibodies) with the desired pharmaceutical carrier(s). Typically, the formulations can be prepared by uniformly and intimately bringing the active ingredients into association with liquid carriers or finely divided solid carriers or both, and then, if necessary, shaping the product. Formulations can be sterilized if desired, provided that the method of sterilization does not interfere with the effectiveness of the antibody(s) contained in the formulation.

Compositions can be formulated into any of many possible dosage forms such as, without limitation, tablets, capsules, liquid syrups, soft gels, suppositories, and enemas. Compositions also can be formulated as suspensions in aqueous, non-aqueous or mixed media. Aqueous suspensions further can contain substances that increase the viscosity of the suspension including, for example, sodium carboxymethyl-cellulose, sorbitol, and/or dextran. Suspensions also can contain stabilizers.

### 5. Methods

The methods provided herein typically include administering to a mammal (e.g., a dog, a cat, a horse, a cow, a rabbit, a rat, a mouse, or a human) a molecule (e.g., an antibody such as sHIgM12) or a composition (e.g., a composition containing sHIgM12) provided herein. In some embodiments, the methods also can include administration of DC that have been contacted with a molecule or a composition provided herein (e.g., a compositions containing sHIgM12 and an antigen). Such DC are useful to potentiate an immune response in the mammal to which they are administered.

As described above, the molecule, composition, or activated DC can be administered by any suitable systemic or local method. Systemic methods of administration include, without limitation, oral, topical, or parenteral administration, as well as administration by injection.

### 6. Articles of manufacture

Articles of manufacture provided herein can include one or more molecules and/or compositions disclosed herein. The molecule and/or composition can be combined with packaging material and sold as kits for treating or reducing development of allergic asthma. The molecule and/or composition can be in a container such as a vial, a tube, or a syringe, for example, and can be at least partially surrounded with packaging material. Components and methods for producing articles of manufacture are well known.

Articles of manufacture may combine one or more of the molecules set out in the above sections. For example, an article of manufacture can contain a composition that includes a molecule provided herein (e.g., an antibody such as sHIgM12 ). An article of manufacture also can include one or more antigens (e.g., a tumor antigen or an antigen from a pathogen) that can stimulate a specific immune response. Furthermore, an article of manufacture can contain DC. An article of manufacture also may include, for example, buffers or other control reagents for potentiating an immune response. Instructions, also can be included in such kits.

The invention will be further described in the following examples, which do not limit the scope of the invention described in the claims.

### EXAMPLES

### Example 1 - Materials and methods for isolating and characterizing sHIgM12

*Isolation of human antibodies -* Human serum samples were obtained from the dysproteinemia clinic, and those exhibiting an Ig clonal peak of greater than 20 mg/ml were chosen for further evaluation. The selected samples were from 50 patients with a wide variety of conditions characterized by a monoclonal IgM spike, including Waldenstrom's macroglobulinemia, lymphoma, and monoclonal gammopathy of undetermined significance. Sera were dialyzed against water, and precipitates were collected by centrifugation at 14,000 rpm for 30 minutes and dissolved in phosphate buffered saline (PBS). The samples were centrifuged and subjected to chromatography on a Superose-6 column (Amersham Pharmacia, Piscataway, NJ). IgM fractions were pooled and analyzed by SDS-PAGE, and protein concentrations were determined by reading absorbance at 280 nm. IgM solutions were sterile filtered and cryopreserved. The antibody sHIgM12 was identified based on its ability to bind DC as determined by FACS analysis. The polyclonal human IgM antibody control was described previously (Miller et al. (1994) J. Neurosci. 14:6230-6238).

Monomeric sHIgM12 was obtained from the pentameric form by reduction with 5 mM dithiothreitol (Sigma-Aldrich, St. Louis, MO) in 200 mM Tris, 150 mM NaCl, 1 mM EDTA pH 8.0 for 2 hours at room temperature. Subsequent alkylation was performed with 12 mM iodacetamide for 1 hour on ice. IgM monomers were isolated by chromatography on a Superdex-200 column (Amersham Pharmacia) equilibrated with PBS, and characterized by reducing and non-reducing SDS-PAGE.

### Example 2 - Production of recombinant human IgM antibodies

Once antibodies of interest such as sHIgM12 are identified, immortalized sources are generated to sustain these important reagents. A vector system was developed and used to immortalize sHIgM12 as well as another human IgM antibody (sHIgM22) identified in the serum of a Waldenstrom's macroglobulinemia patient. The amino acid sequence of the antibodies were determined from Fv fragments generated from the serum. Since malignant B cells circulate in the blood of Waldenstrom patients, cDNA encoding the heavy and light chain genes of the sHIgM22 antibody present in highest serum concentrations was successfully isolated. These cDNA sequences were used to generate a genomic human IgM heavy chain gene encoding the variable region derived from the patient antibody and a cDNA-based light chain gene expressed under control of the cytomegalovirus (CMV) promoter. These antibody gene sequences were incorporated into a single vector (Figure 3) along with a selectable dHfR gene expressed under the control of a SV40 promoter. The vector bearing the synthetic antibody genes was introduced into F3B6 hybridoma cells by electroporation. Methotrexate resistant cells were selected and amplified by stepping up the amount of methotrexate in the culture medium. A clone expressing 100 µg antibody per ml of supernatant was recovered. The recombinant antibody displayed all functional properties identified for the antibody isolated from the patient serum.

This same procedure was used to generate a recombinant supply of sHIgM12. An amino acid sequence analysis of sHIgM12 was obtained. Since the amino-terminus of the antibody heavy chain was blocked, Fv fragments were generated to increase the efficiency of obtaining an amino terminal sequence. The amino terminal sequence of the sHIgM12 heavy chain was determined to be Val-Gln-Leu-Gln-Glu-Ser-Gly-Pro-Gly-Leu-Leu-Lys-Pro-Ser-Glu-Thr-Leu-Arg/Ser-Leu-Thr-Asn (SEQ ID NO:4), while the amino terminal sequence of the light chain was determined to be Asp-Ile-Gln-Met-Thr-Gln-Ser-Pro-Ser-Ser-Leu-Ser-Ala-Ser-Val-Gly-Asp-Arg-Val (SEQ ID NO:5).

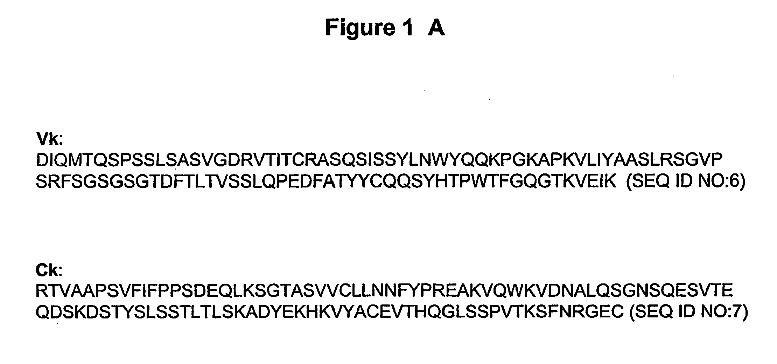

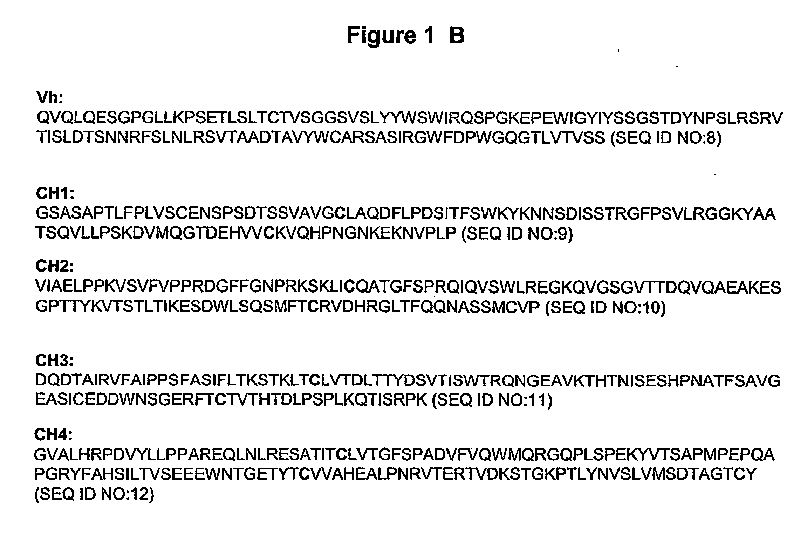

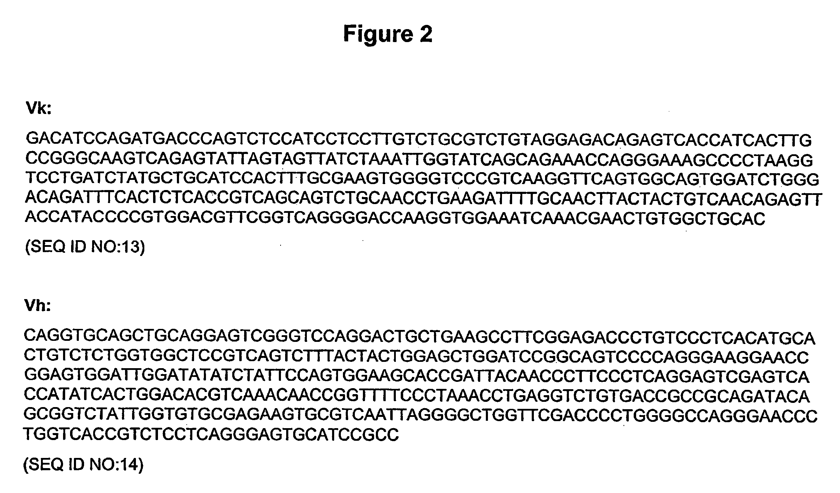

cDNA was isolated from the patient's peripheral blood cells, to be used for recovering full length cDNA copies of the mRNA encoding sHIgM12. In order to ensure than recovered cDNAs truly represented the antibody of interest, the amino acid sequences of CDR3 regions of sHIgM12 were determined. This was accomplished by proteolytic digestion of the Fv fragments and conventional amino acid sequencing of the digestion products. Once the sHIgM12 cDNAs were obtained, they were inserted into a vector that was similar to that described above but was modified for expression of IgM/Kappa antibodies by substituting the light chain constant region. Recombinant sHIgM12 then was expressed in the human/mouse hybridoma line F3B6 as described above. Amino acid sequences for the variable (Vk) and constant (Clc) domains of the sHIgM12 light chain are set forth in SEQ ID NOS:6 and 7, respectively. Amino acid sequences for the variable (Vh) and constant (CH1, CH2, CH3, and CH4) domains of the sHIgM12 heavy chain are set forth, respectively, in SEQ ID NOS:8,9,10,11, and 12. These amino acid sequences also are provided in Figures 1A and 1B. Nucleotide sequences encoding the variable regions of the sHIgM12 light and heavy chains are shown in FIG. 2 (SEQ ID NOS:13 and 14, respectively). The modified vector also was used successfully to express the human antibody rHIgM46.

### OTHER EMBODIMENTS

It is to be understood that while the invention has been described in conjunction with the detailed description thereof, the foregoing description is intended to illustrate and not limit the scope of the invention, which is defined by the scope of the appended claims. Other aspects, advantages, and modifications are within the scope of the following claims.

## Claims

1. A recombinantly produced antibody comprising an amino acid sequence that is at least 95% identical to the amino acid sequence set forth in SEQ ID NO:6 and the amino acid sequence set forth in SEQ ID NO:8.

2. The antibody of claim 1, wherein said antibody further comprises an amino acid sequence that is at least 80.0% identical to the amino acid sequence set forth in SEQ ID NO:7, SEQ ID NO:9, SEQ ID NO:10, SEQ ID NO:11, or SEQ ID NO:12.

3. The recombinantly produced antibody according to claim 1 comprising the amino acid sequence set forth in SEQ ID NO:6 and the amino acid sequence set forth in SEQ ID NO:8.

4. The recombinandy produced antibody according to claim 3, wherein said antibody further comprises an amino acid sequence that is at least 80.0% identical to the amino acid sequence set forth in SEQ ID NO:7, SEQ ID NO:9, SEQ ID NO:10, SEQ ID NO:11, or SEQ ID NO:12.

5. An isolated nucleic acid encoding the antibody of any of claims 1-4.

6. A composition comprising a pharmaceutically acceptable carrier and
(a) the antibody of any one of claims 1-4, or
(b) the nucleic acid molecule of claim 5.

7. The isolated nucleic acid sequence according to claim 5, wherein the nucleic acid sequence comprises the nucleic acid sequence set forth in SEQ ID NO:13 or SEQ ID NO:14.

8. The recombinantly produced antibody according to any one of claims 1-4, wherein the antibody is an IgM antibody.

## Patentansprüche

1. Ein rekombinant hergestellter Antikörper, der eine Aminosäuresequenz umfasst, die mindestens 95% identisch ist mit der Aminosäuresequenz, die in SEQ ID NO:6 dargelegt ist und der Aminosäuresequenz, die in SEQ ID NO:8 dargelegt ist.

2. Der Antikörper von Anspruch 1, wobei genannter Antikörper weiter eine Aminosäuresequenz umfasst, die mindestens zu 80,0% identisch ist mit der Aminosäuresequenz, die in SEQ ID NO:7, SEQ ID NO:9, SEQ ID NO:10, SEQ ID NO:11, oder SEQ ID NO:12 dargelegt ist.

3. Der rekombinant hergestellte Antikörper gemäß Anspruch 1, der die Aminosäuresequenz, die in SEQ ID NO:6 und die Aminosäuresequenz, die in SEQ ID NO:8 dargelegt ist, umfasst.

4. Der rekombinant hergestellte Antikörper gemäß Anspruch 3, wobei genannter Antikörper weiter eine Aminosäuresequenz umfasst, die mindestens zu 80,0% identisch ist mit der Aminosäuresequenz, die in SEQ ID NO:7, SEQ ID NO:9, SEQ ID NO:10, SEQ ID NO:11, oder SEQ ID NO:12 dargelegt ist.

5. Eine isolierte Nukleinsäure, die den Antikörper irgendeines der Ansprüche 1-4 kodiert.

6. Eine Zusammensetzung, welche einen pharmazeutisch akzeptablen Träger und
(a) den Antikörper irgendeines der Ansprüche 1-4, oder
(b) das Nukleinsäuremolekül von Anspruch 5
umfasst.

7. Die isolierte Nukleinsäuresequenz gemäß Anspruch 5, wobei die Nukleinsäuresequenz die Nukleinsäuresequenz, die in SEQ ID NO:13 oder SEQ ID NO:14 dargestellt ist, umfasst.

8. Der rekombinant hergestellte Antikörper gemäß irgendeines der Ansprüche 1-4, wobei der Antikörper ein IgM-Antikörper ist.

## Revendications

1. Anticorps produit de façon recombinante comprenant une séquence d'acides aminés qui est identique à au moins 95 % à la séquence d'acides aminés exposée dans SEQ ID NO : 6 et la séquence d'acides aminés exposée dans SEQ ID NO : 8.

2. Anticorps selon la revendication 1, dans lequel ledit anticorps comprend en outre une séquence d'acides aminés qui est identique à au moins 80 % à la séquence d'acides aminés exposée dans SEQ ID NO : 7, SEQ ID NO : 9, SEQ ID NO : 10, SEQ ID NO : 11 ou SEQ ID NO : 12.

3. Anticorps produit de façon recombinante selon la revendication 1, comprenant la séquence d'acides aminés exposée dans SEQ ID NO : 6 et la séquence d'acides aminés exposée dans SEQ ID NO : 8.

4. Anticorps produit de façon recombinante selon la revendication 3, dans lequel ledit anticorps comprend en outre une séquence d'acides aminés qui est identique à au moins 80 % à la séquence d'acides aminés exposée dans SEQ ID NO : 7, SEQ ID NO : 9, SEQ ID NO : 10, SEQ ID NO : 11 ou SEQ ID NO : 12.

5. Acide nucléique isolé codant pour l'anticorps de l'une quelconque des revendications 1 à 4.

6. Composition comprenant un vecteur pharmaceutiquement acceptable et
(a) l'anticorps de l'une quelconque des revendications 1 à 4, ou
(b) la molécule d'acide nucléique de la revendication 5.

7. Séquence d'acide nucléique isolé selon la revendication 5, dans laquelle la séquence d'acide nucléique comprend la séquence d'acide nucléique exposée dans SEQ ID NO : 13 ou SEQ ID NO : 14.

8. Anticorps produit de façon recombinante selon l'une quelconque des revendications 1 à 4, dans lequel l'anticorps est un anticorps d'IgM.


### SEQUENCE LISTING

<110> Mayo Foundation for Medical Education and Research
<120> B7-DC Binding Antibody
<130> 07039-558WO1
<150> US 10/983,104 <151> 2004-11-05
<150> US 10/881,661 2004-06-30
<160> 14
<170> FastSEQ for Windows Version 4.0
<210> 1
   <211> 8
   <212> PRT
   <213> Gallus-gallus
<400> 1
   Ser Ile Ile Asn Phe Glu Lys Leu
    1               5
<210> 2
   <211> 14
   <212> PRT
   <213> Gallus gallus
<400> 2
   Ile Ser Gln Ala Val His Ala Ala His Ala Glu Ile Asn Glu
    1               5                  10
<210> 3
   <211> 20
   <212> DNA
   <213> Artificial Sequence
<220>
   <223> Synthetically generated oligonucleotide
<400> 3
   tccatgacgt tcctgacgtt 20
<210> 4
   <211> 21
   <212> PRT
   <213> Homo sapiens
<220>
   <221> VARIANT
   <222> 18
   <223> Xaa = Arg or Ser
<400> 4
   Val Gln Leu Gln Glu Ser Gly Pro Gly Leu Leu Lys Pro Ser Glu Thr
    1               5                  10                  15
   Leu Xaa Leu Thr Asn
               20
<210> 5
   <211> 19
   <212> PRT
   <213> Homo sapiens
<400> 5
   Asp Ile Gln Met Thr Gln Ser Pro Ser Ser Leu Ser Ala Ser Val Gly
    1               5                  10                  15
   Asp Arg Val
<210> 6
   <211> 106
   <212> PRT
   <213> Homo sapiens
<400> 6
   Asp Ile Gln Met Thr Gln Ser Pro Ser Ser Leu Ser Ala Ser Val Gly
    1               5                  10                  15
   Asp Arg Val Thr Ile Thr Cys Arg Ala Ser Gln Ser Ile Ser Ser Tyr
               20                  25                  30
   Leu Asn Trp Tyr Gln Gln Lys Pro Gly Lys Ala Pro Lys Val Leu Ile
           35                  40                  45
   Tyr Ala Ala Ser Leu Arg Ser Gly Val Pro Ser Arg Phe Ser Gly Ser
       50                  55                  60
   Gly Ser Gly Thr Asp Phe Thr Leu Thr Val Ser Ser Leu Gln Pro Glu
   65                  70                  75                  80
   Asp Phe Ala Thr Tyr Tyr Cys Gln Gln Ser Tyr His Thr Pro Trp Thr
                   85                  90                  95
   Phe Gly Gln Gly Thr Lys Val Glu Ile Lys
               100                 105
<210> 7
   <211> 107
   <212> PRT
   <213> Homo sapiens
<400> 7
<210> 8
   <211> 118
   <212> PRT
   <213> Homo sapiens
<400> 8
   Gln Val Gln Leu Gln Glu Ser Gly Pro Gly Leu Leu Lys Pro Ser Glu
    1               5                  10                  15
   Thr Leu Ser Leu Thr Cys Thr Val Ser Gly Gly Ser Val Ser Leu Tyr
               20                  25                  30
   Tyr Trp Ser Trp Ile Arg Gln Ser Pro Gly Lys Glu Pro Glu Trp Ile
           35                  40                  45
   Gly Tyr Ile Tyr Ser Ser Gly Ser Thr Asp Tyr Asn Pro Ser Leu Arg
       50                  55                  60
   Ser Arg Val Thr Ile Ser Leu Asp Thr Ser Asn Asn Arg Phe Ser Leu
   65                  70                  75                  80
   Asn Leu Arg Ser Val Thr Ala Ala Asp Thr Ala Val Tyr Trp Cys Ala
                   85                  90                  95
   Arg Ser Ala Ser Ile Arg Gly Trp Phe Asp Pro Trp Gly Gln Gly Thr
               100                 105                 110
   Leu Val Thr Val Ser Ser
           115
<210> 9
   <211> 104
   <212> PRT
   <213> Homo sapiens
<400> 9
   Gly Ser Ala Ser Ala Pro Thr Leu Phe Pro Leu Val Ser Cys Glu Asn
    1               5                  10                  15
   Ser Pro Ser Asp Thr Ser Ser Val Ala Val Gly Cys Leu Ala Gln Asp
               20                  25                  30
   Phe Leu Pro Asp Ser Ile Thr Phe Ser Trp Lys Tyr Lys Asn Asn Ser
           35                  40                  45
   Asp Ile Ser Ser Thr Arg Gly Phe Pro Ser Val Leu Arg Gly Gly Lys
       50                  55                  60
   Tyr Ala Ala Thr Ser Gln Val Leu Leu Pro Ser Lys Asp Val Met Gln
   65                  70                  75                  80
   Gly Thr Asp Glu His Val Val Cys Lys Val Gln His Pro Asn Gly Asn
                   85                  90                  95
   Lys Glu Lys Asn Val Pro Leu Pro
               100
<210> 10
   <211> 112
   <212> PRT
   <213> Homo sapiens
<400> 10
   Val Ile Ala Glu Leu Pro Pro Lys Val Ser Val Phe Val Pro Pro Arg
    1               5                  10                  15
   Asp Gly Phe Phe Gly Asn Pro Arg Lys Ser Lys Leu Ile Cys Gln Ala
               20                  25                  30
   Thr Gly Phe Ser Pro Arg Gln Ile Gln Val Ser Trp Leu Arg Glu Gly
           35                  40                  45
   Lys Gln Val Gly Ser Gly Val Thr Thr Asp Gln Val Gln Ala Glu Ala
       50                  55                  60
   Lys Glu Ser Gly Pro Thr Thr Tyr Lys Val Thr Ser Thr Leu Thr Ile
   65                  70                  75                  80
   Lys Glu Ser Asp Trp Leu Ser Gln Ser Met Phe Thr Cys Arg Val Asp
                   85                  90                  95
   His Arg Gly Leu Thr Phe Gln Gln Asn Ala Ser Ser Met Cys Val Pro
               100                 105                 110
<210> 11
   <211> 106
   <212> PRT
   <213> Homo sapiens
<400> 11
   Asp Gln Asp Thr Ala Ile Arg Val Phe Ala Ile Pro Pro Ser Phe Ala
    1               5                  10                  15
   Ser Ile Phe Leu Thr Lys Ser Thr Lys Leu Thr Cys Leu Val Thr Asp
               20                  25                  30
   Leu Thr Thr Tyr Asp Ser Val Thr Ile Ser Trp Thr Arg Gln Asn Gly
           35                  40                  45
   Glu Ala Val Lys Thr His Thr Asn Ile Ser Glu Ser His Pro Asn Ala
       50                  55                  60
   Thr Phe Ser Ala Val Gly Glu Ala Ser Ile Cys Glu Asp Asp Trp Asn
   65                  70                  75                  80
   Ser Gly Glu Arg Phe Thr Cys Thr Val Thr His Thr Asp Leu Pro Ser
                   85                  90                  95
   Pro Leu Lys Gln Thr Ile Ser Arg Pro Lys
               100                 105
<210> 12
   <211> 131
   <212> PRT
   <213> Homo sapiens
<400> 12
   Gly Val Ala Leu His Arg Pro Asp Val Tyr Leu Leu Pro Pro Ala Arg
    1               5                  10                  15
   Glu Gln Leu Asn Leu Arg Glu Ser Ala Thr Ile Thr Cys Leu Val Thr
               20                  25                  30
   Gly Phe Ser Pro Ala Asp Val Phe Val Gln Trp Met Gln Arg Gly Gln
           35                  40                  45
   Pro Leu Ser Pro Glu Lys Tyr Val Thr Ser Ala Pro Met Pro Glu Pro
       50                  55                  60
   Gln Ala Pro Gly Arg Tyr Phe Ala His Ser Ile Leu Thr Val Ser Glu
   65                  70                  75                  80
   Glu Glu Trp Asn Thr Gly Glu Thr Tyr Thr Cys Val Val Ala His Glu
                   85                  90                  95
   Ala Leu Pro Asn Arg Val Thr Glu Arg Thr Val Asp Lys Ser Thr Gly
               100                 105                 110
   Lys Pro Thr Leu Tyr Asn Val Ser Leu Val Met Ser Asp Thr Ala Gly
           115                 120                 125
   Thr Cys Tyr
       130
<210> 13
   <211> 337
   <212> DNA
   <213> Homo sapiens
<400> 13
   gacatccaga tgacccagtc tccatcctcc ttgtctgcgt ctgtaggaga cagagtcacc    60
   atcacttgcc gggcaagtca gagtattagt agttatctaa attggtatca gcagaaacca   120
   gggaaagccc ctaaggtcct gatctatgct gcatccactt tgcgaagtgg ggtcccgtca   180
   aggttcagtg gcagtggatc tgggacagat ttcactctca ccgtcagcag tctgcaacct   240
   gaagattttg caacttacta ctgtcaacag agttaccata ccccgtggac gttcggtcag   300
   gggaccaagg tggaaatcaa acgaactgtg gctgcac                            337
<210> 14
   <211> 369
   <212> DNA
   <213> Homo sapiens
<400> 14
   caggtgcagc tgcaggagtc gggtccagga ctgctgaagc cttcggagac cctgtccctc    60
   acatgcactg tctctggtgg ctccgtcagt ctttactact ggagctggat ccggcagtcc   120
   ccagggaagg aaccggagtg gattggatat atctattcca gtggaagcac cgattacaac   180
   ccttccctca ggagtcgagt caccatatca ctggacacgt caaacaaccg gttttcccta   240
   aacctgaggt ctgtgaccgc cgcagataca gcggtctatt ggtgtgcgag aagtgcgtca   300
   attaggggct ggttcgaccc ctggggccag ggaaccctgg tcaccgtctc ctcagggagt   360
   gcatccgcc                                                           369